# EUROPÄISCHE PATENTANMELDUNG

(11) **EP 0 547 334 A1**
(43) Veröffentlichungstag der Anmeldung: **23.06.1993**
(21) Anmeldenummer: 92118040.2
(22) Anmeldetag: 22.10.1992
(51) Int. Cl.: A61K 31/66, A61K 31/645, A61K 31/44

(54) **Kombination von Calciumantagonisten mit Cholinesterase-Inhibitoren zur Behandlung von seniler Demenz**

(30) Priorität: 04.11.1991 DE 4136288
(71) Anmelder: Troponwerke GmbH & Co. KG, D-51063 Köln (DE)
(72) Erfinder: de Jonge, Maarten, Dr., W-5063 Overath (DE)
(74) Vertreter: Dänner, Klaus, Dr.

(57) **Zusammenfassung**

Die gedächnisverbessernde Wirkung von Cholinesterase-Inhibitoren kann durch Kombination mit Calciumantagonisten signifikant verbessert werden. Die Kombinationen können dabei in Arzneimittel zur Behandlung von seniler Demenz, insbesondere vom Alzheimer Typ, verwendet werden.

## Beschreibung

Die Erfindung betrifft Kombinationen von Calciumantagonisten mit Cholinesterase-Inhibitoren, Verfahren zur Herstellung und ihre Verwendung in Arzneimitteln zur Verbesserung der Gedächtnisleistung und anderer kognitiven Funktionen bei seniler Demenz und ähnlichen Erkrankungen.

Senile Demenz, insbesondere vom Typ des Morbus Alzheimer, ist eine degenerative Erkrankung des Gehirns, bei der vor allem cholinerge Neuronen absterben [M. Altavista et. al., Brain Research 508, 51 bis 59 (1990); S. Lehericy et. al., Proc. Natl. Acad. Sci., USA, 86, 8580 bis 8584 (1989): K. Oyanagi et. al., Brain Research 504, 354 bis 357 (1989)]. Dies hat ein Acetylcholin-Defizit in verschiedenen Gehirnstrukturen zur Folge, welches mitverantwortlich für die kognitiven Störungen ist [W. Fischer et. al., European Journal of Neuroscience 1(1), 34 bis 45 (1989)].

Es ist bekannt, daß durch die Verabreichung von Cholinesterase-Inhibitoren wie Physostigmin oder Metrifonat, die Aktivität des cholinergen Systems im Gehirn erhöht und damit die kognitive Leistung verbessert werden kann [Becker et. al., Drug Development Research 19, 425 bis 434 (1990); dto. 12, 163 bis 195 (1988)].

Calciumantagonisten sind Substanzen, die den Einstrom von Calcium in die Zelle verringern. Es ist bekannt, daß bestimmte Calciumantagonisten, insbesondere vom Typ der Dihydropyridine, in der Lage sind, die kognitive Leistung von alten Versuchstieren und von alten Menschen mit Hirnleistungsstörungen zu verbessern [T. Bann et. al., Pro. Neuro-Psychopharmacol. and Biol. Psychiat. 14, 525 bis 551 (1990); M. Sandin et. al., Neurobiol. of Aging 11, 573 bis 575 (1990)].

Es wurde nun gefunden, daß Calciumantagonisten (Komponente A) die gedächtnisverbessernde Wirkung von Cholinesterase-Inhibitoren (Komponente B) überraschenderweise in synergistischer Weise verstärken.

Unter Calciumantagonisten (Komponente A) im Rahmen der Erfindung werden in erster Linie solche der Dihydropyridin-Klasse verstanden

Bevorzugt sind Calciumantagonisten der allgemeinen Formel (I), 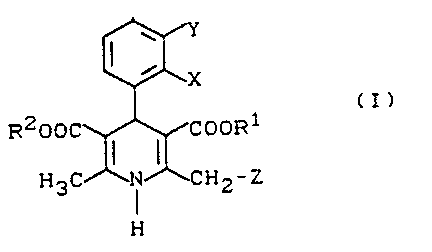
in welcher
- X und Y: gleich oder verschieden sind und für Wasserstoff, Nitro oder Halogen stehen, oder
- X und Y: gemeinsam mit dem Phenylring einen Benzoxadiazolylrest bilden,
- R¹ und R²: gleich oder verschieden sind und für geradkettiges oder verzweigtes Alkyl mit bis zu 6 Kohlenstoffatomen stehen, das durch Alkoxy mit bis zu 4 Kohlenstoffatomen, eine N-Benzyl-N-methyl-aminogruppe oder eine 4-(Diphenylmethyl)-piperazinogruppe substituiert sein kann, oder
für eine Gruppe der Formel 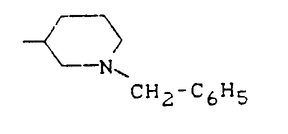 stehen,
und
- Z: für Wasserstoff oder für die Gruppe -O-CH₂CH₂-NH₂ steht.

Besonders bevorzugt sind die Dihydropyridine der allgemeinen Formel (I), in welcher
- X und Y: gleich oder verschieden sind und für Wasserstoff, Nitro oder Chlor stehen,
- R¹ und R²: gleich oder verschieden sind und für geradkettiges oder verzweigtes Alkyl mit bis zu 4 Kohlenstoffatomen stehen, das durch Methoxy substituiert sein kann und
- Z: für Wasserstoff steht.

Insbesondere zu nennen sind:
a) Nimodipin [X = H, Y = NO₂, Z = H, R¹ = Isopropyl, R² = Methyloxyethyl]
b) Nitrendipin [X = H, Y = NO₂_{,} Z = H, R¹ = Methyl, R² = Ethyl]
c) Nisoldipin (X = NO₂, Y = H, Z = H, R¹ = Methyl, R² = Isobutyl]
d) Nifedipin [X = NO₂, Y = H, Z = H, R¹ = Methyl, R² = Methyl]
Unter Cholinesterase-Inhibitoren (Komponente B) im Rahmen der Erfindung werden in erster Linie Stoffe verstanden, die den Abbau von Acetylcholin verhindern.

Bevorzugt sind dies Phosphorverbindungen der allgemeinen Formel (II) oder Stickstoffverbindungen der Formeln (III), (IV) und (V) 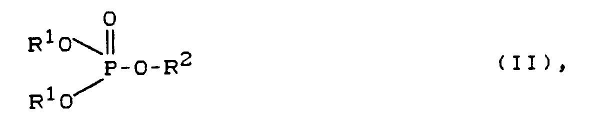 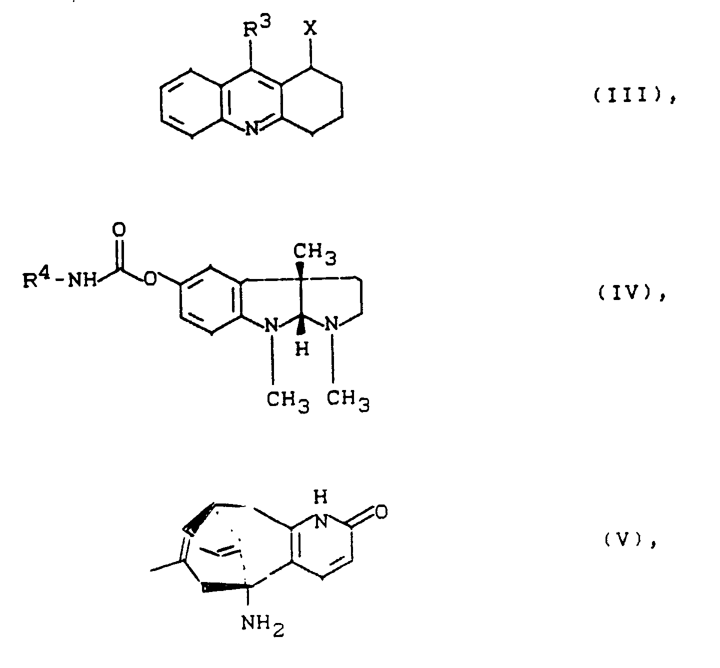
in welchen
- R¹ -: für Alkyl mit bis zu 4 Kohlenstoffatomen steht,
- R² -: für eine Gruppe der Formel 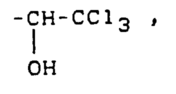 -CH=CCl₂ oder -CH=CBr₂ steht,
- R³ -: für eine Amino- oder Benzylaminogruppe steht,
- R⁴ -: für Alkyl mit bis zu 7 Kohlenstoffatomen steht
und
- X -: für Wasserstoff oder Hydroxy steht.

Besonders bevorzugt sind Verbindungen der Formel (II), in welcher
- R¹ -: für Methyl steht und
- R² -: für eine Gruppe der Formel 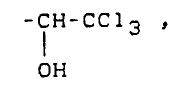 -CH=CCl₂ oder -CH=CBr₂ steht.

Durch die Kombination mit Calciumantagonisten wird die Wirkung der Cholinesterase-Inhibitoren signifikant verbessert. Dies ist vor allem bei schlecht verträglichen, beispielsweise lebertoxischen Cholinesterase- Inhibitoren von besonderem Vorteil, da die Behandlungsdosis verringert werden kann und damit weniger Nebenwirkungen auftreten.

Die als Komponente A eingesetzten Dihydropyridine sind an sich bekannt [US 3 799 934; US 4 154 839; US 3 485 847; DE 2 407 115; US 4 264 611; US 4 572 909; US 4 466 972; US 4 338 322].

Die als Komponente B eingesetzten Cholinesterase-Inhibitoren sind ebenfalls bekannt [US 2 701 225; US 2 956 073].

Der folgende Versuch belegt beispielhaft den unerwarteten synergistischen, überadditiven Effekt der erfindungsgemäßen Kombination:

### Testmethode - passiver Vermeidungstest

Eine Woche vor Testbeginn werden Ratten in eine geräuschundurchdringliche Kammer, in der alle Tests vorgenommen werden, gebracht. Während des Versuchsdurchgangs 1 wird jedes Tier auf eine kleine schwarze Plattform gesetzt, die durch eine 60 W starke Glühbirne hell erleuchtet ist. Die Plattform hat einen Zugang zu einem dunkleren Abschnitt mit einem Gitterboden. Die Latenz bis zum Betreten des dunklen Abschnitts wird gemessen. In dieser Phase des Versuches werden die Tiere an die Testsituation gewöhnt.

Vier Stunden später, während des 2. Versuchsdurchgangs (Schockversuch), werden die Tiere beim Betreten des dunklen Abschnitts für 2 Sekunden einem 220 µA starken elektrischen Schock an den Füßen ausgesetzt. Substanzen werden nach folgendem Schema an Ratten (10-12 pro Gruppe) oral in Volumen von 2 ml/kg appliziert:

| 45 Minuten vor dem Schockversuch | 30 Minuten vor dem Schockverusch | Gruppe |
|---|---|---|
| Kontrolle (H₂O) | Kontrolle (1 % Methylzellulose) | I |
| Metrifonat (1 mg/kg) | Kontrolle (1 % Methylzellulose) | II |
| Kontrolle (H₂O) | Nimodipin (0,1-10,0 mg/kg po) | III |
| Metrifonat (1 mg/kg) | Nimodipin (0,1-10,0 mg/kg po) | IV |

Es ist bekannt, daß Metrifonat in dieser Dosierung (1 mg/kg) keine Wirkung hat in diesem Test [B. Schmidt, M. de Jonge, Biol. Psychiatry 29, 486s (1991)]. Pro Versuch wird eine Dosierung Nimodipin (0,1; 0,5; 1; 5 und 10 mg/kg po in 1 % Methylzellulose) geprüft.

Vierundzwanzig Stunden nach dem Schockversuch (3. Versuchsdurchgang, sogen. Retentionsversuch) wird der 1. Versuchsdurchgang wiederholt.

Für alle Versuchsdurchgänge wird eine maximale Vermeidungs-Latenz von 180 Sekunden erlaubt.

Die Differenzen in den Latenzwerten zwischen Kontroll- und Experiment-Gruppen, die beim Retentionsversuch auftreten, werden nach dem Mann-Whitney U-Test und einer Varianzanalyse statistisch evaluiert. Unterschiede in der Anzahl der Tiere, die den dunklen Abschnitt nicht betreten (maximale Vermeidungslatenz von 180 Sekunden), werden unter Verwendung des Fischer-Exact-Probability-Tests statistisch bewertet.

Wenn im Retentionsversuch Tiere der Gruppe IV im Vergleich mit Tieren aus den Kontrollgruppen I bis III entweder längere Zeit bis zum Betreten des dunklen Abschnitts benötigen oder diesen überhaupt nicht betreten, so zeigt dies, daS die Tiere, denen die erfindungsgemäße Kombination appliziert wurde, eine bessere Erinnerung an den Schockversuch, vierundzwanzig Stunden zuvor, besitzen, d.h., daß die Kombination mit Calciumantagonisten den Kognitions-verbessernden Effekt von Cholinesterase-Inhibitoren synergistisch steigert.

Bezogen auf einen Gewichtsteil des Cholinesterase-Inhibitors (Komponente B) können 0,01 bis 100 Gewichtsteile, bevorzugt 0,1 bis 10 Gewichtsteile, des Calciumantagonisten (Komponente A) eingesetzt werden.

Besonders gute Wirkung zeigt die Kombination von Metrifonat mit Nimodipin, insbesondere im Verhältnis 1 zu 10.

Die Kombination kann hergestellt werden, indem man die Einzelkomponenten in diese auflösende inerte Lösemittel auflöst und gegebenenfalls nach Abdampfen des Lösemittels die Kombination in üblicher Weise mit Hilfsstoffen vermischt. Als inerte Lösemittel seien beispielhaft Alkohole wie Ethanol oder Polyethylenglykol genannt. Die Komponenten können auch als Feststoff gemischt werden.

Zur vorliegenden Erfindung gehören auch pharmazeutische Zubereitungen, die neben inerten, nichttoxischen, pharmazeutisch geeigneten Hilfs- und Trägerstoffen die erfindungsgemäße Kombination enthalten, die aus der erfindungsgemäßen Kombination bestehen, sowie Verfahren zur Herstellung dieser Zubereitungen.

Die Kombination soll in diesen Zubereitungen in einer Konzentration von 0,1 bis 99,5 Gew.-%, bevorzugt von 0,5 bis 95 Gew.-%, der Gesamtmischung vorhanden sein.

Neben der Kombination können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, die Kombination in Gesamtmengen von etwa 0,01 bis etwa 100 mg/kg, bevorzugt in Gesamtmengen von etwa 1 mg/kg bis 50 mg/kg, Körpergewicht je 24 Stunden, gegebenenfalls in Form mehrerer Einzelgaben zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament der Art und Schwere der Erkrankung, der Art der Zubereitung und Applikation, sowie dem Zeitpunkt bzw. Intervall, zu welchem die Verabreichung erfolgt.

## Patentansprüche

1. Kombination bestehend aus Calciumantagonisten (als Komponente A) udn Cholinesterase-Inhibitoren (als Komponente B).

2. Kombination nach Anspruch 1, dadurch gekennzeichnet, daß als Komponente A Calciumantagonisten der allgemeinen Formel 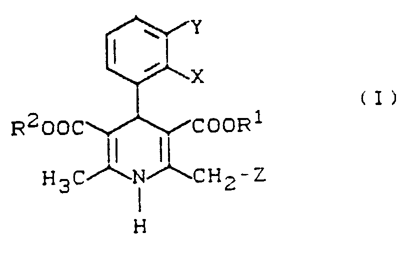 in welcher
X und Y gleich oder verschieden sind und für Wasserstoff, Nitro oder Halogen stehen, oder
X und Y gemeinsam mit dem Phenylring einen Benzoxadiazolylrest bilden,
R¹ und R² gleich oder verschieden sind und für geradkettiges oder verzweigtes Alkyl mit bis zu 6 Kohlenstoffatomen stehen, das durch Alkoxy mit bis zu 4 Kohlenstoffatomen, eine N-Benzyl-N-methyl-aminogruppe oder eine 4-(Diphenylmethyl)-piperazinogruppe substituiert sein kann, oder
für eine Gruppe der Formel 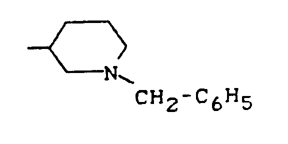
und
Z für Wasserstoff oder für die Gruppe -O-CH₂CH₂-NH₂ steht,
eingesetzt werden.

3. Kombination nach Anspruch 1, dadurch gekennzeichnet, daß als Komponente A Calciumantagonisten der Formel I, in welcher
X und Y gleich oder verschieden sind und für Wasserstoff, Nitro oder Chlor stehen,
R¹ und R² gleich oder verschieden sind und für geradkettiges oder verzweigtes Alkyl mit bis zu 4 Kohlenstoffatomen stehen, das durch Methoxy substituiert sein kann und
Z für Wasserstoff steht.

4. Kombination nach Anspruch 1, dadurch gekennzeichnet, daß als Komponente A Nimodipin, Nitrendipin, Nisoldipin oder Nifedipin eingesetzt wird.

5. Kombination nach Anspruch 1, dadurch gekennzeichnet, daß als Komponente B Phosphorverbindungen der allgemeinen Formel (II) oder Stickstoffverbindungen der Formeln (III), (IV) und (V) 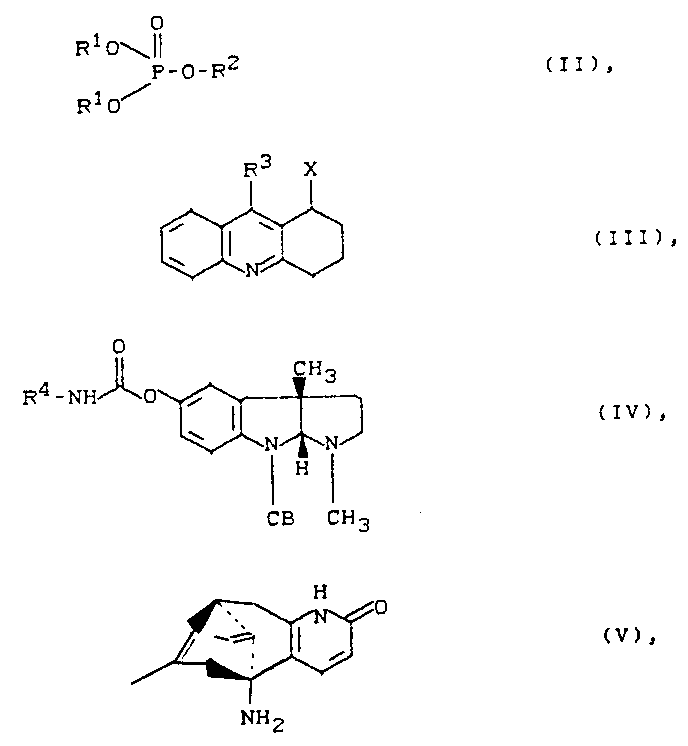 in welchen
R¹ - für Alkyl mit bis zu 4 Kohlenstoffatomen steht,
R² - für eine Gruppe der Formel 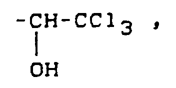 -CH=CCl₂ oder -CH=CBr₂ steht,
R³ - für eine Amino- oder Benzylaminogruppe steht, und
X - für Wasserstoff oder Hydroxy steht,
eingesetzt wird.

6. Kombination nach Anspruch 1, dadurch gekennzeichnet, daß als Komponente B Phosphorverbindungen der Formel II, in welchen
R¹ für Methyl steht und
R² für eine Gruppe der Formel 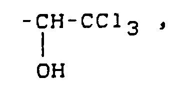 -CH=CCl₂ oder -CH=CBr₂ steht,
eingesetzt werden.

7. Kombination nach Anspruch 1 zur Verbesserung der Gedächnisleistung bei seniler Demenz.

8. Kombination nach Anspruch 1, enthaltend auf 1 Gewichtsteil der Komponente B 0,01 bis 100 Gewichtsteile der Komponente A.

9. Verwendung der Kombination nach Anspruch 1 zur Herstellung von Arzneimitteln zur Verbesserung der Gedächnisleistung bei seniler Demenz.

10. Verfahren zur Herstellung der Kombination nach Anspruch 1, dadurch gekennzeichnet, daß man die Einzelkomponenten in diese auflösenden inerten Lösemittel auflöst und gegebenenfalls nach Abdampfen des Lösemittels die Kombination in üblicher Weise mit Hilfsstoffen vermischt.
